Europäisches Patentamt

European Patent Office

Office européen des brevets

(11) Veröffentlichungsnummer: **0 521 258 A1**

## (12) EUROPÄISCHE PATENTANMELDUNG

(21) Anmeldenummer: **92107480.3**

(22) Anmeldetag: **02.05.92**

(51) Int. Cl.5: **C07H 15/04**

(30) Priorität: **04.07.91 DE 4122071**

(43) Veröffentlichungstag der Anmeldung:
**07.01.93 Patentblatt 93/01**

(84) Benannte Vertragsstaaten:
**DE ES FR GB IT**

(71) Anmelder: **HÜLS AKTIENGESELLSCHAFT**
**Patentabteilung / PB 15 - Postfach 13 20**
**W-4370 Marl 1(DE)**

(72) Erfinder: **Ripke, Norbert, Dr.**
**Hellweg 28**
**W-4358 Haltern(DE)**

(54) **Verfahren zur Neutralisation von Alkylpolyglycosid-Lösungen.**

(57) Bei der Herstellung von Alkylpolyglycosiden fallen zuletzt saure, nichtwäßrige Lösungen der Produkte in langkettigen Alkoholen an.

Die Erfindung betrifft ein neues Verfahren zur Neutralisation dieser Lösungen. Dabei wird zunächst ein kleiner Teil der Lösung entnommen und mit einem Gemisch aus Alkohol mit 1 bis 6 C-Atomen und Wasser versetzt, worauf mit wäßrigem, alkoholischem oder wäßrig-alkoholischem Alkali neutralisiert wird. Anschließend wird die Hauptmenge der Alkylpolyglycosid-Lösungmit der berechneten Menge Alkali neutralisiert.

Die Erfindung betrifft ein Verfahren zur Neutralisation von sauren Lösungen von Alkylpolyglycosiden mit Alkylgruppen mit 8 bis 24 C-Atomen in Alkoholen mit 8 bis 24 C-Atomen.

Alkylpolyglycoside sind ungiftige und leicht abbaubare oberflächenaktive Stoffe. Sie werden deshalb als Wasch- und Reinigungsmittel und als Emulgatoren und Dispergatoren verwendet. Sie haben aber nur dann die gewünschten Grenzflächeneigenschaften, wenn die Alkylgruppen mindestens 8 C-Atome aufweisen.

Alkylglycoside und Alkylpolyglycoside mit langkettigen Alkylgruppen werden im allgemeinen durch mehrstufige Synthesen hergestellt. So wird beispielsweise in EP-A-0 306 652 ein zweistufiges Verfahren angegeben, wonach man zunächst durch Glycosidierung mit n-Butanol ein n-Butylglycosid und daraus durch Umglycosidierung mit einem langkettigen Alkohol das gewünschte langkettige Alkylpolyglycosid herstellt. Beide Reaktionen, Glycosidierung und Umglycosidierung, werden durch Säuren katalysiert. Nach beendeter Umglycosidierung wird neutralisiert, worauf überschüssiger langkettiger Alkohol destilliert wird.

Es sind auch einstufige Reaktionen zur Herstellung von Alkylpolysacchariden bekannt. Dabei werden beispielsweise nach US 3 839 318 Sacharide unter speziellen Reaktionsbedingungen direkt mit langkettigen Alkoholen umgesetzt. Auch hier muß nach beendeter Reaktion neutralisiert werden, worauf überschüssiger langkettiger Alkohol abdestilliert wird.

Nach EP-A-0 249 013 kann der Polymerisationsgrad von Alkylpolyglycosiden bei der Umglycosidierung mit Hilfe der Säuremenge beeinflußt werden. Nach beendeter Umglycosidierung wird hier neutralisiert. Dabei verwendet man eine der angewendeten Menge Schwefelsäure äquivalente Menge an Natronlauge. Für eine präzise Einstellung bestimmter pH-Werte ist diese Methode zu ungenau, da sie Verluste an Säure, die beispielsweise durch Veresterungen entstehen können, unberücksichtigt läßt.

Da sich Saccharide im sauren Medium leicht thermisch zersetzen, muß die vorhandene Säure vor der Destillation der überschüssigen Alkohole zumindest weitgehend neutralisiert werden. Dabei besteht die Schwierigkeit, daß die Messung des pH-Wertes in einer fettalkoholischen Lösung nicht direkt möglich ist. Es bestand daher die Aufgabe, eine Methode zu entwickeln, nach der saure Lösungen von Alkylpolyglycosiden in langkettigen Alkoholen schnell und genau neutralisiert und auf bestimmte pH-Werte eingestellt werden können.

Die Aufgabe wird erfindungsgemäß dadurch gelöst, daß man einen kleinen Teil der Lösung entnimmt, ihn mit der 2- bis 10fachen Menge einer Mischung aus 50 bis 90 Gewichtsprozent Alkohol mit 1 bis 6 C-Atomen und 50 bis 10 Gewichtsprozent Wasser versetzt und ihn dann mit Alkali, das als wäßrige, alkoholische oder wäßrig-alkoholische Lösung zugesetzt wird, neutralisiert. Anschließend wird die Hauptmenge der Alkylpolyglycosid-Lösung mit der hier ermittelten Menge an Alkali neutralisiert.

Neutralisation bedeutet hier nicht nur, daß ein pH-Wert von 7 eingestellt wird. Im Sinne dieser Erfindung bedeutet Neutralisation, daß die vorhandene Säure zumindest weitgehend neutralisiert wird. Andererseits kann dabei auch übertitriert werden, so daß man schwach alkalische Lösungen erhält. Die hier durchgeführten Neutralisationen können also zu Lösungen mit pH-Werten von 6,5 bis 11 führen. Vorzugsweise wird ein pH-Wert von 7 bis 10 eingestellt.

Unter einem kleinen Teil der Alkylpolyglycosid-Lösung wird hier ein Anteil von bis zu 20 % verstanden. Dabei reicht es im allgemeinen völlig aus, wenn man in 0,1 bis 5 % der Lösung die zur Neutralisation benötigte Alkalimenge bestimmt.

Das erfindungsgemäße Verfahren wird vorzugsweise auf kontinuierlich anfallende Alkylpolyglycosid-Lösungen angewandt. Dabei wird ein Seitenstrom abgezweigt. Man bestimmt in ihm die für einen gewünschten pH-Wert benötigte Menge an Alkali, worauf dann der Hauptstrom gezielt mit einer berechneten Menge Alkali behandelt wird.

Im allgemeinen setzt man 10- bis 60%ige Alkylpolyglycosid-Lösungen ein. Dabei werden 15- bis 35%ige Lösungen vorzugsweise verwendet.

Der Zuckeranteil der Alkylpolyglycoside kann von einem Monosaccharid, wie beispielsweise Glucose, Mannose, Gulose, Galaktose oder Talose, aber auch von einem Di- oder einem Oligosaccharid hergeleitet werden. Die Saccharideinheiten können 1,2-, 1,3-, 1,4- oder 1,6-verknüpft sein. Es können $\alpha$- oder $\beta$-Verknüpfungen vorliegen. Die Ketten können linear oder verzweigt sein. Der mittlere Polymerisationsgrad liegt meist im Bereich von 1 bis 10. Der Zuckeranteil leitet sich vorzugsweise von Glucose ab.

Die Alkylreste der Alkylpolyglycoside sind im allgemeinen die gleichen, die auch die Alkohole, in denen die Alkylpolyglycoside gelöst sind, aufweisen.

Geeignete Alkohole mit 8 bis 24 C-Atomen sind beispielsweise Octanol, Nonanol, Decanol, 10-Undecen-1-ol, Dodecanol, Tetradecanol oder Stearylalkohol. Die Alkohole können linear sein. Sie können aber auch Verzweigungen enthalten. Man kann natürliche oder synthetische Alkohole oder Alkoholgemische einsetzen.

Die Alkylreste der Alkylpolyglycoside und die der als Lösemittel verwendeten Alkohole weisen vorzugsweise Kohlenstoffketten mit 10 bis 18 C-Atomen auf.

Die Lösungen der Alkylpolyglycoside enthalten noch die zur Umglycosidierung oder - bei einstufigen Herstellverfahren - die zur Glycosidierung benötigten sauren Katalysatoren. Diese Katalysatoren können Mineralsäuren, wie Schwefel- oder Phosphorsäure, oder auch organische Säuren, wie beispielsweise Aryl-, Alkyl- oder Aralkylsulfonsäuren, sein. Die Säurezahl dieser Lösungen liegt meist im Bereich von 1 bis 10 mg KOH/g. Dabei werden Lösungen mit Säurezahlen von 1,5 bis 5 mg KOH/g bevorzugt eingesetzt.

Die zugesetzte Alkohol/Wasser-Mischung enthält vorzugsweise 60 bis 80 Gewichtsprozent Alkohol und 40 bis 20 Gewichtsprozent Wasser. Für diese Mischung geeignete Alkohole sind beispielsweise Methanol, Ethanol, n-Propanol, Isopropanol, n-Butanol, Isobutanol, n-Pentanol oder n-Hexanol. Alkohole mit 2 bis 4 C-Atomen werden dabei bevorzugt eingesetzt.

Als Alkali kommen vor allem Ammoniak, Amine, Alkali- und Erdalkalihydroxide in Betracht. Vorzugsweise werden Alkalihydroxide, wie z. B. Natrium- und Kaliumhydroxid, eingesetzt.

Die Neutralisation der abgezweigten Lösung und die der Hauptlösung werden im allgemeinen bei 20 bis 90 °C vorgenommen.

Das vorliegende Verfahren ermöglicht eine Bestimmung der zur Neutralisation benötigten Alkalimenge innerhalb weniger Sekunden. Von der Zugabe des Alkohol/Wasser-Gemisches bis zur Alkalizugabe zur Hauptlösung vergehen mitunter nur 30 Sekunden bis 2 Minuten. Die Methode ist so schnell, daß sie gut bei kontinuierlichen Verfahren "on stream" angewandt werden kann. Ein im Seitenstrom bei der Neutralisation erhaltenes Signal kann dabei sofort auf den Hauptstrom übertragen werden, wo die genaue Alkalimenge zudosiert wird.

Die erfindungsgemäße Methode führt zu einer Zeitersparnis und ermöglicht verbesserte Raum-Zeit-Ausbeuten.

Beispiele von Mischungen, die mit dem abgezweigten Teil der Alkylpolyglycosid-Lösungen hergestellt werden, sind in Tabelle 1 angegeben.

## Tabelle 1

| Mischung | | a | b | c | d |
|---|---|---|---|---|---|
| Alkylpolyglycosid-Lösung[1] | [g] | 10 | 10 | 10 | 10 |
| n-Butanol | [g] | 30 | 30 | 50 | - |
| Isopropanol | [g] | - | - | - | 30 |
| Wasser | [g] | 10 | 12 | 30 | 20 |

[1] Zuckeranteil: Glucose, Alkyl : Dodecyl, 25%ige Lösung in Dodecanol

Bei der Neutralisation dieser Lösungen mit wäßrigem 0,1 N NaOH nimmt die Titrationskurve im Äquivalenzbereich einen steilen Verlauf, so daß die für einen gewünschten pH-Wert benötigte NaOH-Menge genau bestimmt werden kann.

## Patentansprüche

1. Verfahren zur Neutralisation von sauren Lösungen von Alkylpolyglycosiden mit Alkylgruppen mit 8 bis 24 C-Atomen in Alkoholen mit 8 bis 24 C-Atomen,
dadurch gekennzeichnet,
daß man einen kleinen Teil der Lösung entnimmt, ihn mit der 2 bis 10fachen Menge einer Mischung aus 50 bis 90 Gewichtsprozent Alkohol mit 1 bis 6 C-Atomen und 50 bis 10 Gewichtsprozent Wasser versetzt und ihn dann mit wäßrigem, alkoholischem oder wäßrig-alkoholischem Alkali neutralisiert,

EP 0 521 258 A1

worauf die Hauptmenge der Alkylpolyglycosid-Lösung mit der berechneten Menge Alkali neutralisiert wird.

2. Verfahren nach Anspruch 1, bei dem die saure Alkylpolyglycosid-Lösung ein kontinuierlicher Strom ist, aus dem ein kleiner Seitenstrom abgezweigt wird.

3. Verfahren nach Anspruch 1,
   dadurch gekennzeichnet,
   daß eine 10- bis 60%ige saure Alkylpolyglycosid-Lösung eingesetzt wird.

4. Verfahren nach Anspruch 1,
   dadurch gekennzeichnet,
   daß eine Mischung aus 60 bis 80 Gewichtsprozent Alkohol und 40 bis 20 Gewichtsprozent Wasser zugesetzt wird.

5. Verfahren nach Anspruch 1,
   dadurch gekennzeichnet,
   daß der Alkohol der zugesetzten Mischung 2 bis 4 C-Atome aufweist.

6. Verfahren nach Anspruch 1,
   dadurch gekennzeichnet,
   daß bei der Neutralisation ein pH-Wert von 7 bis 10 eingestellt wird.

4

| EINSCHLÄGIGE DOKUMENTE | | | EP 92107480.3 |
|---|---|---|---|

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| A | WO - A - 90/07 516 (HENKEL) * Beispiel 1 * -- | 1-6 | C 07 H 15/04 |
| A | EP - A - 0 328 959 (BASF) * Spalte 3, Zeilen 25-48 * ---- | 1,6 | |

RECHERCHIERTE SACHGEBIETE (Int. Cl⁵)

C 07 H 15/00

Der vorliegende Recherchenbericht wurde für alle Patentansprüche erstellt.

| Recherchenort | Abschlußdatum der Recherche | Prüfer |
|---|---|---|
| WIEN | 14-10-1992 | IRMLER |